# DEMANDE DE BREVET EUROPEEN

(11) **EP 2 990 033 A1**
(43) Date de publication de la demande: **02.03.2016**
(21) Numéro de dépôt: 14306318.8
(22) Date de dépôt: 26.08.2014
(51) Int. Cl.: A61K 9/14, A61K 9/16

(54) **Procédé de préparation de nanoprécipites de peptide ou protéine de faible poids moléculaire**

(71) Demandeur: Carlina Technologies, 49100 Angers (FR)
(72) Inventeur: Meyer, Olivier, 49000 Angers (FR); Perrier, Thomas, 49124 Saint Barthelemy D'Anjou (FR); Gonnet, Maud, 49000 Angers (FR)
(74) Mandataire: Regimbeau

(57) **Abrégé**

La présente invention concerne un procédé de préparation non dénaturant de nanoprécipités de peptides ou protéines ou de nanocoprécipités de peptides ou protéines et d'ions métalliques, dans lequel ladite protéine ou ledit peptide est de poids moléculaire inférieur à 15 kDa, avantageusement inférieur à 10 kDa, plus avantageusement inférieur à 8 kDa. Ledit procédé comprend une étape de préparation d'un mélange d'une solution aqueuse de peptides ou protéines, d'un non-solvant du peptide ou de la protéine, et éventuellement un sel métallique hydrosoluble. La présente invention concerne également un nanoprécipité susceptible d'être obtenu par le procédé selon l'invention, ainsi qu'une composition pharmaceutique le comprenant, pour leur utilisation dans le traitement ou la prévention du diabète.

## Description

La présente invention concerne un procédé de préparation non dénaturant de nanoprécipités de peptides ou protéines ou de nanocoprécipités de peptides ou protéines et d'ions métalliques, de préférence d'ions métalliques divalents, dans lequel les peptides ou protéines sont de faible poids moléculaire, ainsi que les nanoprécipités ou nanocoprécipités susceptibles d'être obtenus par un tel procédé. La présente invention concerne également une composition pharmaceutique, en particulier une composition pharmaceutique à libération contrôlée, comprenant lesdits nanoprécipités ou nanocoprécipités ainsi que son utilisation dans les domaines de santé humaine et animale.

Les peptides et protéines sont des structures complexes très organisés, très sensibles à leur environnement et pouvant être facilement dénaturées. La dénaturation d'un peptide ou d'une protéine entraine le plus souvent une perte définitive ou temporaire de son activité. Or, les méthodes de précipitation de peptides ou protéines connues jusqu'à ce jour sont dénaturantes, parce que comprenant des conditions potentiellement néfastes pour la structure des peptides/protéines qui perdent ainsi leur activité, telles que des températures élevées, une forte agitation ou une mise en contact avec des surfaces hydrophobes, des interfaces aqueuses/organiques ou des détergents.

La nanoprécipitation de peptides ou protéines, en particulier de peptides ou protéines thérapeutiques, permet notamment d'améliorer leur profil de libération et leur biodisponibilité dans l'organisme récepteur. Elle permet également la lyophilisation de suspensions concentrées de peptides/protéines et la préparation de composition pharmaceutique fortement concentrée en peptides ou protéines tout en étant peu ou non visqueuse, comprenant les nanoprécipités obtenus en l'absence d'agrégation dénaturante.

Il existe dans la littérature des procédés de nanoprécipitation de protéines, tels que les techniques de désolvatation des protéines, conduisant à l'obtention de précipités de protéines de tailles généralement supérieures au micromètre. Cependant, ces procédés conduisent le plus souvent à la dénaturation des protéines, ou ne permettent pas l'obtention d'un précipité de protéine sans qu'aucun polymère ne soit nécessaire en plus du non-solvant.

Par exemple, la demande de brevet WO 2009/043874 décrit un procédé de préparation de nanoparticules de protéine-poloxamer ayant un diamètre moyen de 50 à 200 nm et dans lesquelles les protéines ont un poids moléculaire de préférence supérieure à 8 kDa et sont non dénaturées. Lesdites nanoparticules sont des complexes protéine-poloxamer, un polymère supplémentaire autre que le non-solvant est donc nécessaire pour les obtenir.

Il existe donc un besoin de développer une technique de nanoprécipitation non dénaturante de peptides ou protéines de faible poids moléculaire, non complexées avec une autre molécule favorisant sa précipitation ou coprécipité(e)s avec un ion métallique, de préférence d'ions métalliques divalents. Les peptides ou protéines sont de préférence des peptides ou protéines thérapeutiques, tels que l'insuline par exemple.

Dans le cadre de la présente invention, les inventeurs ont découvert un nouveau procédé de préparation non dénaturant de nanoprécipités de peptides ou protéines de poids moléculaire inférieur à 15 kDa, avantageusement inférieur à 10 kDa, plus avantageusement inférieur à 8 kDa, permettant d'obtenir des nanoprécipités de peptides ou protéines non dénaturé(e)s, ou des nanocoprécipités desdits peptides ou desdites protéines non dénaturé(e)s et d'ions métalliques, de préférence d'ions métalliques divalents, pouvant être incorporées dans une composition pharmaceutique, de préférence un composition pharmaceutique à libération contrôlée.

La présente invention permet d'obtenir des nanoprécipités de peptides ou protéines de faible poids moléculaire, ou de nanocoprécipités de peptides ou protéines de faible poids moléculaire et d'ions métalliques, dans des conditions douces non-dénaturantes. Elle consiste à mettre en contact une solution de peptides ou protéines de faible poids moléculaire, un non-solvant organique des peptides ou protéines, tel que les diols organiques choisis parmi les polyéthylène glycols ou dérivés de polyéthylène glycols de faible poids moléculaire, notamment le PEG 550 et les diols organiques choisis dans le groupe de l'hexylène glycol, butane-1,4-diol, pentane-1,5-diol, étohexadiol, 2-méthylpentane-2,4-diol(hexylène glycol), 3-cyclopentene-1,2-diol, cis-4-cyclopentene-1,3-diol, trans-1,4-dioxane-2,3-diol, 1,3-dioxane-5,5-dimethanol, (3S,4S)-pyrrolidine-3,4-diol, (3R,4R)-(-)-1-benzyl-3,4-pyrrolidinediol, (3S,4S)-(+)-1-benzyl-3,4-pyrrolidinediol, 3-cyclopentene-1,2-diol, 2-methyl-butane-1,3-diol.

Le procédé de nanoprécipitation selon l'invention permet l'obtention de nanoprécipités ou nanocoprécipités de peptides ou protéines pouvant être utilisé(e)s en tant que médicament à administration unique parentérale de telle sorte que la durée pendant laquelle la concentration plasmatique desdits peptides ou protéines est dans la fenêtre thérapeutique est supérieure à la durée pendant laquelle la concentration plasmatique après une administration unique parentérale des mêmes peptides ou protéines non préparé(e)s selon le procédé de préparation selon l'invention est dans la fenêtre thérapeutique. Ainsi, la nanoprécipitation selon l'invention permet de contrôler la vitesse de libération des peptides ou protéines, notamment pour obtenir une libération prolongée du peptide ou de la protéine dans une forme biologiquement active pendant au moins 2 jours, de préférence de 2 jours à plusieurs mois.

La nanoprécipitation selon l'invention permet également la préparation de composition pharmaceutique fortement concentrée en lesdits peptides ou protéines tout en étant peu visqueuse. La faible viscosité d'une composition pharmaceutique est particulièrement utile pour son administration par voie parentérale, en particulier pour son administration parentérale non intraveineuse.

Dans un premier aspect, la présente invention concerne donc un procédé de préparation non dénaturant de nanoprécipités de peptides ou protéines ou de nanocoprécipités de peptides ou protéines et d'ions métalliques, ayant un diamètre moyen inférieur à 1 µm, comprenant les étapes suivantes :
a) préparation d'un mélange d'une solution aqueuse de peptides ou protéines, d'un non-solvant des peptides ou protéines, et éventuellement un sel métallique hydrosoluble;
b) agitation douce du mélange obtenu à l'étape a) ;
c) séparation solide-liquide du mélange obtenu à l'étape b) ; et
d) éventuellement, récupération des nanoprécipités de peptides ou protéines ou de nanocoprécipités de peptides ou protéines et d'ions métalliques,
dans lequel lesdits peptides ou protéines sont de poids moléculaire inférieur à 15 kDa, avantageusement inférieur à 10 kDa, plus avantageusement inférieur à 8 kDa,
et ledit non-solvant est choisi parmi les polyéthylène glycols ou dérivés de polyéthylène glycols de poids moléculaire inférieur à 2000 Da, avantageusement compris entre 200 et 2000 Da, plus avantageusement de 550 Da, et les diols organiques choisis dans le groupe de l'hexylène glycol, butane-1,4-diol, pentane-1,5-diol, étohexadiol, 2-méthylpentane-2,4-diol(hexylène glycol), 3-cyclopentene-1,2-diol, cis-4-cyclopentene-1,3-diol, trans-1,4-dioxane-2,3-diol, 1,3-dioxane-5,5-dimethanol, (3S,4S)-pyrrolidine-3,4-diol, (3R,4R)-(-)-1-benzyl-3,4-pyrrolidinediol, (3S,4S)-(+)-1-benzyl-3,4-pyrrolidinediol, 3-cyclopentene-1,2-diol, 2-methyl-butane-1,3-diol.

Dans le cadre de la présente invention, on entend par « procédé de préparation non dénaturant », un procédé de nanoprécipitation ou nanocoprécipitation de peptides ou protéines qui ne dénature pas lesdits peptides ou protéines. Le procédé de nanoprécipitation ou nanocoprécipitation selon l'invention permet donc l'obtention de peptides ou protéines conservant leur conformation tridimensionnelle normale ainsi que leur intégrité et leur activité biologique. De manière préférée, le procédé selon l'invention permet d'obtenir des nanoprécipités ou nanocoprécipités de peptides ou protéines dans lesquels 85 %, en particulier 90 %, plus particulièrement 100%, des protéines ou peptides sont non dénaturé(e)s. De manière davantage préférée, le procédé selon l'invention permet d'obtenir des nanoprécipités ou nanocoprécipités de protéines ou peptides dans lesquels 100%, des protéines ou peptides sont non dénaturé(e)s.

Dans le cadre de l'invention, on entend par « nanoprécipité de peptides ou protéines », des particules de diamètre moyen inférieur à 1 µm, avantageusement compris entre 5nm et 500 nm, plus avantageusement compris entre 5 nm et 200 nm, encore plus avantageusement compris entre 5 nm et 170 nm, en particulier compris entre 5 nm et 150 nm, qui sont le résultat de la précipitation d'un ou plusieurs peptides et/ou d'une ou plusieurs protéines dans un non-solvant. Les nanoprécipités de peptides ou protéines selon l'invention consistent essentiellement en le ou les peptides ou la ou les protéines.

Selon un mode de réalisation avantageux, les nanoprécipités de peptides ou protéines selon l'invention consistent en plus de 90 %, en particulier plus de 95%, plus particulièrement plus de 99% de peptide(s) ou protéine(s), le reste étant des impuretés, par exemple des impuretés de non-solvant utilisé.

Avantageusement, la présente invention concerne un procédé de préparation non dénaturant de nanoprécipités de peptides ou protéines de faible poids moléculaire ou de nanocoprécipités de peptides ou protéines de faible poids moléculaire et d'ions métalliques, ayant un diamètre moyen inférieur à 1 µm, avantageusement compris entre 5nm et 500 nm, plus avantageusement compris entre 5 nm et 200 nm, encore plus avantageusement compris entre 5 nm et 170 nm, en particulier compris entre 5 nm et 150 nm.

Selon un autre aspect, la présente invention concerne un procédé de préparation non dénaturant de nanocoprécipités de peptides ou protéines et d'ions métalliques, dans lequel lesdits peptides ou protéines sont de poids moléculaire inférieur à 15 kDa, avantageusement inférieur à 10 kDa, plus avantageusement inférieur à 8 kDa, et ledit ion métallique est choisi parmi le zinc (Zn), le manganèse (Mn), le magnésium (Mg), le calcium (Ca), le fer (Fe), le lithium (Li) et le cuivre (Cu). De préférence, ledit ion métallique est un ion métallique divalent choisi parmi le zinc (Zn), le manganèse (Mn), le magnésium (Mg), le calcium (Ca), le fer (Fe) et le cuivre (Cu), de manière préférée le zinc (Zn) ou le manganèse (Mn).

Par « ion métallique monovalent », on entend les ions ayant une valence de un et qui, par conséquent, peuvent former des liaisons avec un autre ion ou molécule. Par exemple, un anion métallique monovalent est un ion ayant un électron supplémentaire par rapport à son état élémentaire. De même, un cation métallique monovalent est un ion ayant un électron manquant par rapport à son état élémentaire.

De même, par « ion métallique divalent », on entend les ions ayant une valence de deux et qui, par conséquent, peuvent former des liaisons avec deux autres ions ou molécules. Par exemple, un anion métallique divalent est un ion ayant deux électrons supplémentaire par rapport à son état élémentaire. De même, un cation métallique divalent est un ion ayant deux électrons manquant par rapport à son état élémentaire.

On entend par « nanocoprécipité de peptides ou protéines et d'ions métalliques » encore appelé « nanocoprécipité de peptides ou protéines/ion métallique », le résultat de la co-précipitation d'une ou plusieurs protéine(s) ou peptide(s) avec un ion métallique. Ces nanocoprécipités consistent donc essentiellement en le ou les peptides ou la ou les protéines, ledit ion métallique et éventuellement un résidu du non-solvant utilisé.

Par « peptide ou protéine de faible poids moléculaire », on entend les protéines, ou peptides de poids moléculaire inférieur à 15 kDa, avantageusement inférieur à 10 kDa, plus avantageusement inférieur à 8 kDa.

Par non-solvant organique du peptide ou de la protéine, on entend un solvant dans lequel la protéine ou le peptide n'est pas soluble, ce qui entraine la formation d'un précipité, de préférence d'un nanoprécipité.

Font partie des « non solvants » selon l'invention, les diols organiques choisis parmi les polyéthylène glycols ou dérivés de polyéthylène glycols de poids moléculaire inférieur à 2000 Da, avantageusement compris entre 200 et 2000 Da, plus avantageusement de 500 Da, et les diols organiques choisis dans le groupe de l'hexylène glycol, butane-1,4-diol, pentane-1,5-diol, étohexadiol, 2-méthylpentane-2,4-diol(hexylène glycol), 3-cyclopentene-1,2-diol, cis-4-cyclopentene-1,3-diol, trans-1,4-dioxane-2,3-diol, 1,3-dioxane-5,5-dimethanol, (3S,4S)-pyrrolidine-3,4-diol, (3R,4R)-(-)-1-benzyl-3,4-pyrrolidinediol, (3S,4S)-(+)-1-benzyl-3,4-pyrrolidinediol, 3-cyclopentene-1,2-diol, 2-methyl-butane-1,3-diol. De manière préférée, le non-solvant du peptide ou de la protéine selon l'invention est choisi parmi le PEG 550, le glycofurol et l'hexylène glycol ((±)-2-Methyl-2,4-pentanediol), de préférence parmi le PEG 550 et l'hexylène glycol. De manière encore plus préférée, le non-solvant du peptide ou de la protéine selon l'invention est le PEG 550.

Dans le cadre de la présente invention, les termes « polyéthylène glycols ou dérivés de polyéthylène glycols de faible poids moléculaire » représentent des diols organiques choisis parmi les polyéthylène glycols ou dérivés de polyéthylène glycols de poids moléculaire inférieur à 2000 Da, avantageusement compris entre 200 et 2000 Da, plus avantageusement de 500 Da, et l'hexylène glycol. Les polyéthylène glycols ou dérivés de polyéthylène glycols de faible poids moléculaire selon l'invention sont choisi parmi PEG 100, PEG 200, PEG 300, PEG 400, PEG 550, PEG 600, PEG 900, PEG 1000, PEG 2000, le glycofurol. De manière préférée, les polyéthylène glycols ou dérivés de polyéthylène glycols de faible poids moléculaire selon l'invention sont choisi parmi le PEG 550 et le glycofurol, de préférence le polyéthylène glycol de faible poids moléculaire selon l'invention est le PEG 550.

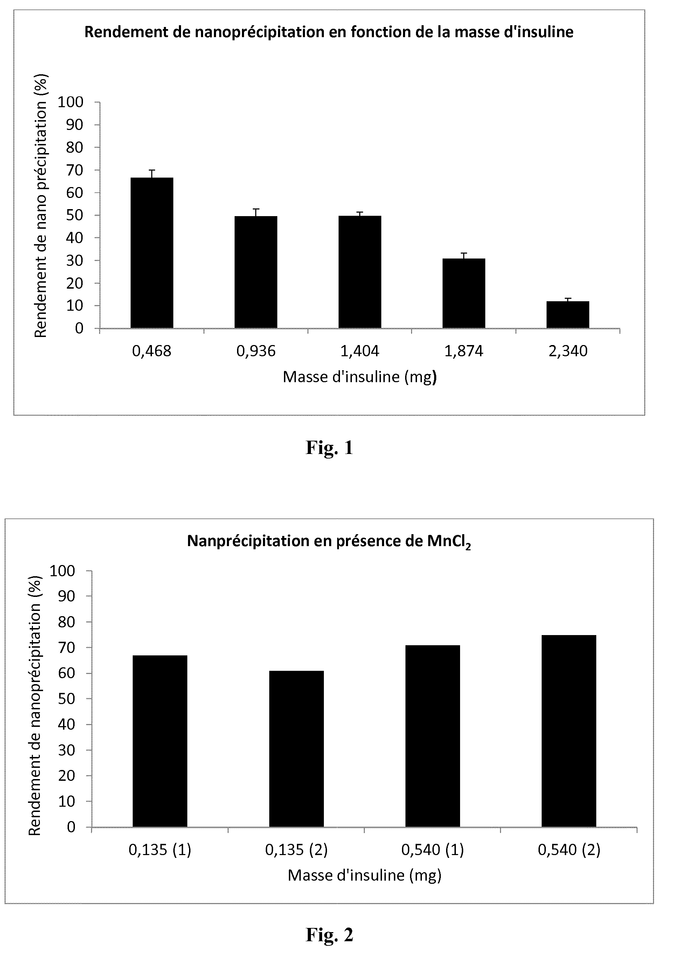

Dans le cadre de la présente invention, le non-solvant du peptide ou de la protéine est utilisé dans une quantité suffisante pour précipiter lesdits peptides ou protéines, ou nanocoprécipiter lesdits peptides ou protéines et l'ion métallique, sous forme de nanoparticules ayant un diamètre moyen inférieur à 1 µm. Le rapport volumique solution de peptides ou protéines / non-solvant des peptides ou des protéines peut être déterminé selon des méthodes connues de l'homme du métier. Le rendement de la nanoprécipitation ou nanocoprécipitation dépend particulièrement de la quantité des peptides ou protéines à nanoprécipiter ou nanocoprécipiter. En effet, comme le démontre la Figure 1, plus la quantité de peptides ou protéines à nanoprécipiter ou nanocoprécipiter est faible, plus la nanoprécipitation ou nanocoprécipitation est facile et plus le rendement est élevé. Une augmentation de la quantité de peptides ou protéines à nanoprécipiter ou nanocoprécipiter peut entrainer une baisse du rendement.

De manière préférée, la présente invention concerne un procédé de préparation non dénaturant de nanoprécipités de peptides ou protéines thérapeutiques ou de nanocoprécipités de peptides ou protéines thérapeutiques / ions métalliques.

On entend par « peptide ou protéine thérapeutique », tout peptide ou toute protéine dont l'administration permet le traitement d'une ou plusieurs pathologies. Parmi les peptides ou protéines thérapeutiques, on peut citer les enzymes, les cytokines, les facteurs de croissance, les hormones, les anticorps et les facteurs de coagulation, ainsi que les agents de diagnostic, notamment les hormones peptidiques telles que insulines et dérivés, glucagon et analogues (glucagon-like peptides), hormones de croissance, somatostatines, vasopressines, calcitonines, LHRH (Luteinizing-hormone-releasing-hormone) agonistes et antagonistes, ACTH (hormone adrénocorticotrope), peptides synergiques de l'ACTH, hormone somatotropique, hormone lutéotrope, hormone tyréotrope, hormone foliculo-stimulante, hormone stimulante de cellules interstitielles (ICSH), thyréolibérine, corticolibérine, somatolibérine, lutéolubérine, facteur d'inhibition de la prolactine, tyrocidine A, penicillines, gramicidines, ocytocine, peptides vaccins, et leurs dérivés naturels et synthétiques ou leurs fragments. Avantageusement, la présente invention concerne un procédé de préparation non dénaturant de nanoprécipités de peptides ou protéines choisis parmi l'insuline humaine, l'hormone de croissance, le glucagon, les hormones peptidiques ou un de leurs dérivés ou fragments thérapeutiquement efficaces. Plus avantageusement la présente invention concerne un procédé de préparation non dénaturant de nanoprécipités d'insuline humaine ou d'un de ses dérivés ou fragments thérapeutiquement efficaces ou d'un nanocoprécipité d'insuline humaine ou d'un de ses dérivés ou fragments thérapeutiquement efficaces et d'ions métalliques, de préférence un nanocoprécipité d'insuline humaine / zinc.

Les termes « dérivés thérapeutiquement efficaces » d'un peptide ou d'une protéine, couvrent les peptides ou protéines dans lesquels un ou plusieurs résidus d'acides aminés ont été substitués par d'autres résidus d'acides aminés et/ou dans lesquels un ou plusieurs résidus d'acides aminés du peptide ou de la protéine ont été supprimés et/ou dans lesquels un ou plusieurs résidus d'acides aminés ont été ajoutés ou au peptide ou à la protéine. Les termes « dérivés thérapeutiquement efficaces » d'un peptide ou d'une protéine couvrent également les peptides ou protéines PEGylés, glycosylés et acétylés.

Par « fragment thérapeutiquement efficace» d'un peptide ou d'une protéine, on entend une partie du polypeptide, du peptide ou de la protéine qui est plus courte que la longueur du peptide ou de la protéine à l'état naturelle et qui a une activité thérapeutique efficace. Par exemple, par « fragment thérapeutiquement efficace de l'insuline », on entend une partie du polypeptide d'insuline qui est plus courte que la longueur de la protéine d'insuline à l'état naturelle et qui a une activité thérapeutique efficace, en particulier contre le diabète.

Selon la présente invention, la nanoprécipitation de peptides ou protéines ou la nanocoprécipitation de peptides ou protéines et d'ions métalliques se produit dans une large gamme de température, notamment à une température comprise entre -20 et 37 °C, plus particulièrement à une température comprise entre 0 et 10 °C.

Dans un mode de réalisation préféré, l'agitation douce est effectuée selon les méthodes connues de l'homme du métier, plus particulièrement par agitation mécanique ou magnétique (<50 tour/min.)

Dans un autre mode de réalisation préféré, l'étape b) du procédé selon l'invention, est suivie d'une étape durant laquelle le mélange est refroidi, avantageusement dans un bain de glace, pendant 1 minute à 60 minutes à une température comprise entre 0 °C et 10 °C.

Dans un encore autre mode de réalisation préféré, la séparation solide/liquide de l'étape c) du procédé selon l'invention est une centrifugation, en particulier dans une gamme de force centrifuge comprise entre environ 500 et 50000 G. De même, l'étape d) de récupération des nanoprécipités est effectuée par filtration sur membrane ou filtration tangentielle.

La nanoprécipitation peut éventuellement être réalisée en présence de sel métallique hydrosoluble, tels que ZnCl₂, MgCl₂, CaCl₂, MnCl₂, FeCl₂, CuSO₄ ou LiCl. De préférence, le sel métallique hydrosoluble est le chlorure de zinc ZnCl₂ ou le chlorure de manganèse MnCl₂. On parle alors de nanocoprécipitation selon l'invention.

Avantageusement, l'utilisation d'un sel hydrosoluble en combinaison avec un non-solvant des peptides ou protéines favorise et/ou améliore la précipitation des peptides ou protéines, par la formation de complexes ion métallique/protéine (ou peptide). Le sel permet notamment d'obtenir de meilleurs rendements de précipitation tout en conservant l'activité des peptides ou protéines après précipitation et re-dissolution.

La concentration en sel de la solution aqueuse peut varier dans un large intervalle. Pour une quantité donnée de peptides ou protéines, à un pH de la solution fixe, une température fixe et un rapport de volume fixe d'eau / non-solvant miscible à l'eau, l'homme du métier peut déterminer une concentration minimale en sel appropriée par un travail de routine, typiquement en ajoutant des quantités croissantes de sel jusqu'à l'observation de la précipitation des peptides ou protéines. Cependant, lorsque la nanoprécipitation est réalisée en présence de sel hydrosoluble, le rendement de la nanocoprécipitation dépend de la quantité de peptides ou protéines et de la concentration en sel. Comme le démontrent les Figures 2, 3, 4 et 5, lorsque la quantité de peptides ou protéines est faible, le rendement de la nanocoprécipitation dépend fortement de la concentration en sel et il augmente avec la diminution de la concentration en sel.

Ainsi, le procédé selon l'invention peut être caractérisé en ce que ledit mélange de l'étape a) comprend également au moins un sel métallique hydrosoluble choisi parmi le ZnCl₂, MgCl₂, CaCl₂, MnCl₂, FeCl₂, LiCl et le CuSO₄, avantageusement le ZnCl₂ ou le MnCl₂.

L'invention concerne en outre un nanoprécipité de protéines ou peptides ou un nanocoprécipités de peptides ou protéines et d'ions métalliques, dans lequel lesdits peptides ou protéines sont de poids moléculaire inférieur à 15 kDa, avantageusement inférieur à 10 kDa, plus avantageusement inférieur à 8 kDa, susceptible d'être obtenu par le procédé selon l'invention.

De préférence, les nanoprécipités ou nanocoprécipités selon l'invention sont constitués de plus de 85 %, en particulier plus de 90 %, plus particulièrement 100%, de protéines ou peptides non dénaturé(e)s.

De préférence, les nanoprécipités ou nanocoprécipités selon l'invention ont un diamètre moyen inférieur à 1 µm, avantageusement compris entre 5 nm et 500 nm, plus avantageusement compris entre 5 nm et 200 nm, encore plus avantageusement compris entre 5 nm et 170 nm, en particulier compris entre 5 nm et 150 nm. Ils sont récupérés afin, notamment, d'être incorporés dans une préparation pharmaceutique adéquate.

Plus particulièrement, les nanoprécipités ou nanocoprécipités de peptides ou protéines selon l'invention peuvent être utilisés en tant que médicament à libération prolongée pour contrôler la délivrance in vivo desdits peptides ou protéines.

Dans un autre aspect, l'invention concerne des nanoprécipités de peptides ou protéines ou des nanocoprécipités de peptides ou protéines et d'ions métalliques selon l'invention, pour leur utilisation en tant que médicament.

L'invention concerne également des nanoprécipités de peptides ou protéines ou des nanocoprécipités de peptides ou protéines et d'ions métalliques selon l'invention, pour leur utilisation en tant que médicament à administration unique parentérale, en particulier par voie parentérale non intraveineuse, de telle sorte que la durée pendant laquelle la concentration plasmatique desdits peptides ou protéines se trouve dans la fenêtre thérapeutique est supérieure à la durée pendant laquelle la concentration plasmatique après une administration unique parentérale, en particulier par voie parentérale non intraveineuse, des mêmes peptides ou protéines non préparé(e)s selon le procédé de préparation selon l'invention se trouve dans la fenêtre thérapeutique.

Dans le cadre de la présente invention, on entend par « fenêtre thérapeutique » d'un principe actif, l'ensemble des concentrations plasmatiques dudit principe actif comprises entre la concentration plasmatique minimale thérapeutique dudit principe actif, c'est-à-dire la concentration plasmatique minimale à partir de laquelle le principe actif présente une activité thérapeutique efficace, et la concentration plasmatique maximale thérapeutique dudit principe actif, c'est-à-dire la concentration plasmatique maximale dudit principe actif pouvant être atteinte sans entrainer d'effets secondaires gênants.

En particulier, l'invention concerne des nanoprécipités de peptides ou protéines ou des nanocoprécipités de peptides ou protéines et d'ions métalliques selon l'invention, pour leur utilisation en tant que médicament à administration unique parentérale, en particulier par voie parentérale non intraveineuse, de telle sorte que la durée pendant laquelle la concentration plasmatique desdits peptides ou protéines se trouve dans la fenêtre thérapeutique est supérieure à 2 jours, de préférence supérieure à 7 jours, de manière préférée supérieure à 21 jours, de manière davantage préférée supérieure à 30 jours.

Plus particulièrement, l'invention concerne des nanoprécipités de peptides ou protéines ou des nanocoprécipités de peptides ou protéines et d'ions métalliques selon l'invention, pour leur utilisation en tant que médicament à administration unique parentérale, en particulier par voie parentérale non intraveineuse, de telle sorte que la durée pendant laquelle la concentration plasmatique desdits peptides ou protéines se trouve dans la fenêtre thérapeutique est supérieure à 21 jours, de manière davantage préférée supérieure à 30 jours.

Sous un autre aspect, l'invention concerne des nanoprécipités de peptides ou protéines ou des nanocoprécipités de peptides ou protéines et d'ions métalliques selon l'invention, pour leur utilisation en tant que médicament à administration unique parentérale, en particulier par voie parentérale non intraveineuse, de telle sorte que la durée pendant laquelle la concentration plasmatique desdits peptides ou protéines se trouve dans la fenêtre thérapeutique est supérieure de 2 jours, de préférence supérieure de 7 jours, de manière préférée supérieure de 21 jours, de manière davantage préférée supérieure de 30 jours, à la durée pendant laquelle la concentration plasmatique après une administration unique parentérale, en particulier par voie parentérale non intraveineuse, des mêmes peptides ou protéines non préparé(e)s selon le procédé de préparation selon l'invention se trouve dans la fenêtre thérapeutique.

En particulier, l'invention concerne des nanoprécipités de peptides ou protéines ou des nanocoprécipités de peptides ou protéines et d'ions métalliques selon l'invention, pour leur utilisation en tant que médicament à administration unique parentérale, en particulier par voie parentérale non intraveineuse, de telle sorte que la durée pendant laquelle la concentration plasmatique desdits peptides ou protéines se trouve dans la fenêtre thérapeutique est supérieure de 21 jours, de manière davantage préférée supérieure de 30 jours, à la durée pendant laquelle la concentration plasmatique après une administration unique parentérale, en particulier par voie parentérale non intraveineuse, des mêmes peptides ou protéines non préparé(e)s selon le procédé de préparation selon l'invention se trouve dans la fenêtre thérapeutique..

Sous encore un autre aspect, l'invention concerne des nanoprécipités de peptides ou protéines purs ou des nanocoprécipités de peptides ou protéines et d'ions métalliques selon l'invention, pour leur utilisation en tant que médicament à administration unique parentérale, en particulier par voie parentérale non intraveineuse, entre Tₘₐₓ et Tₘₐₓ₊₂ jours, de préférence entre Tₘₐₓ et Tₘₐₓ₊₇ jours, de manière préférée entre Tₘₐₓ et Tₘₐₓ₊₂₁ jours, de manière encore préférée entre Tₘₐₓ et Tₘₐₓ₊₃₀ jours.

Plus particulièrement, après l'administration unique parentérale, en particulier par voie parentérale non intraveineuse, de nanoprécipités ou nanocoprécipités selon l'invention, la concentration plasmatique en peptide ou protéine est de préférence supérieure à 90 % et de manière davantage préférée supérieure à 98 %, de la valeur de la concentration plasmatique maximale (Cₘₐₓ) entre Tₘₐₓ et T_{max+2 jours}, de préférence entre Tₘₐₓ et Tₘₐₓ₊₇ jours, de manière préférée entre Tₘₐₓ et Tₘₐₓ₊₂₁ jours, de manière plus préférée entre Tₘₐₓ et T_{max+30 jours}.

Le terme « concentration plasmatique maximale (Cₘₐₓ) » désigne le pic de concentration représentant le point ou la concentration plasmatique est la plus élevée de toute la cinétique. Ce pic est atteint lorsque les quantités absorbées et éliminées sont égales, il est mesuré après une prise d'une dose unique.

« Tₘₐₓ » est la valeur du temps nécessaire pour atteindre la concentration plasmatique maximale. Cette valeur est indicative de la vitesse d'absorption d'une substance pharmaceutiquement active.

« Tₘₐₓ₊ₓ jours » correspond au temps nécessaire pour atteindre la concentration plasmatique maximale Tₘₐₓ auquel on ajoute x jours, x correspondant à un nombre entier, notamment x est égal à 2, 7, 21 ou 30. Par exemple, « Tₘₐₓ₊₂ jours » correspond à la valeur du temps Tₘₐₓ auquel on ajoute 2 jours.

Dans un aspect préféré de la présente invention, les inventeurs ont ajusté le temps pendant lequel la concentration plasmatique doit être maintenue supérieure à au moins 85 % de la valeur de Cₘₐₓ en fonction du principe actif utilisé. Par exemple et de manière non limitative, des nanoprécipités d'insuline purs ou des nanocoprécipités insuline/zinc selon l'invention peuvent être utilisés en tant que médicament libérant de l'insuline administrée une seule fois par voie parentérale, en particulier par voie parentérale non intraveineuse, de telle sorte que la concentration plasmatique en insuline est supérieure à 85 %, de manière préférée supérieure à 90 %, de manière davantage préférée supérieure à 98 %, de la valeur de Cₘₐₓ entre Tₘₐₓ et Tₘₐₓ₊₂ jours, de préférence entre Tₘₐₓ et Tₘₐₓ₊₅ jours, en particulier entre Tₘₐₓ et Tₘₐₓ₊₇ jours. De même, des nanoprécipités d'hormones de croissance purs ou de nanocoprécipités hormone de croissance/ion métallique selon l'invention sont utilisés en tant que médicament libérant l'hormone de croissance administrée une seule fois par voie parentérale, en particulier par voie parentérale non intraveineuse, de telle sorte que la concentration plasmatique en hormone de croissance est supérieure à 85 %, de manière préférée supérieure à 90 %, de manière davantage préférée supérieure à 98 %, de la valeur de Cₘₐₓ entre Tₘₐₓ et Tₘₐₓ₊₂₁ jours, de préférence entre Tₘₐₓ et Tₘₐₓ₊₃₀ jours.

Dans un mode de réalisation particulier, les nanoprécipités ou nanocoprécipités selon l'invention permettent la libération in vivo desdits peptides ou protéines de manière prolongée, pendant au moins 2 jours, avantageusement au moins 5 jours, plus avantageusement au moins 30 jours, encore plus avantageusement pendant 90 jours. De manière avantageusement préférée, les nanoprécipités ou nanocoprécipités selon l'invention permettent la libération in vivo desdits peptides ou protéines de manière prolongée pendant au moins 7 jours, avantageusement pendant au moins 21 jours, encore plus avantageusement pendant 30 jours.

Les nanoprécipités ou nanocoprécipités selon l'invention sont compatibles avec l'utilisation de tout système ou toute matrice polymérique permettant de relarguer la protéine ou le peptide de façon contrôlée dans le temps. Ainsi, dans un mode de réalisation préféré, les nanoprécipités ou nanocoprécipités selon l'invention sont incorporés dans des matrices polymériques permettant également leur libération prolongée in vivo.

Dans le cadre de la présente invention, on entend par « matrice polymérique », un réseau de polymère, éventuellement tridimensionnel lorsque les polymères sont réticulés, tel qu'un gel. De manière préférée, les matrices polymériques comprennent notamment les polymères biocompatibles, naturels ou synthétiques, choisis parmi les polymères à base d'acide glycolique, d'acide lactique, d'acide pluronique, de polyéthylène glycol, de polysaccharides tels que l'acide hyaluronique, de polypeptides, les copolymères triblock PEO-PPO (poly(ethylene oxide) - poly(propylene oxide)), à base d'isopropylacrylamide, les matrices lipidiques, les dendrimères ou leur mélange. De manière préférée, la matrice polymérique est biodégradable.

Plus particulièrement, la matrice polymérique est un gel, notamment un gel injectable, plus particulièrement un gel thermosensible ou une matrice lipidique, encore plus particulièrement un gel d'acide hyaluronique.

Par « nanoprécipités ou nanocoprécipités incorporés dans des matrices polymérique », on entend des nanoprécipités ou nanocoprécipités selon l'invention qui sont encapsulés, dispersés, greffés ou réticulés, dans la matrice polymérique.

La présente invention concerne également une composition pharmaceutique comprenant des nanoprécipités ou nanocoprécipités selon l'invention, et avantageusement un excipient pharmaceutiquement acceptable. Avantageusement, la composition pharmaceutique selon l'invention comprend les nanoprécipités ou nanocoprécipités selon l'invention incorporés dans des matrices polymériques.

Selon un mode de réalisation avantageux, la composition pharmaceutique selon l'invention est à libération prolongée. Plus particulièrement, la composition selon l'invention libère de manière prolongée les peptides ou protéines pendant plus de 2 jours, avantageusement plus de 5 jours, plus avantageusement plus de 30 jours, encore plus avantageusement plus de 90 jours.

La composition pharmaceutique est particulièrement adaptée pour une administration par voie parentérale, de préférence non intraveineuse, orale, sublinguale, nasale, transdermique, topique, rectale, oculaire et mucosale, de préférence par voie parentérale non intraveineuse. De manière avantageusement préférée, les nanoprécipités ou nanocoprécipités selon l'invention permettent la libération in vivo des peptides ou protéines de manière prolongée pendant 7 jours, avantageusement pendant 21 jours, plus avantageusement pendant 30 jours.

Dans un mode de réalisation préféré, la composition pharmaceutique selon l'invention libère les protéines ou peptides selon l'invention après une administration unique parentérale, en particulier par voie parentérale non intraveineuse de telle sorte que la durée pendant laquelle la concentration plasmatique desdits peptides ou protéines se trouve dans la fenêtre thérapeutique est supérieure à la durée pendant laquelle la concentration plasmatique après une administration unique parentérale, en particulier par voie parentérale non intraveineuse, des mêmes peptides ou protéines non préparé(e)s selon le procédé de préparation selon l'invention se trouve dans la fenêtre thérapeutique.

En particulier, la composition pharmaceutique selon l'invention libère les protéines ou peptides selon l'invention après une administration unique parentérale, en particulier par voie parentérale non intraveineuse, de telle sorte que la durée pendant laquelle la concentration plasmatique desdits peptides ou protéines se trouve dans la fenêtre thérapeutique est supérieure à 2 jours, de préférence supérieure à 7 jours, de manière préférée supérieure à 21 jours, de manière davantage préférée supérieure à 30 jours.

En particulier, la composition pharmaceutique selon l'invention libère les protéines ou peptides selon l'invention après une administration unique parentérale, en particulier par voie parentérale non intraveineuse, de telle sorte que la durée pendant laquelle la concentration plasmatique desdits peptides ou protéines se trouve dans la fenêtre thérapeutique est supérieure de 2 jours, de préférence supérieure de 7 jours, de manière préférée supérieure de 21 jours, de manière davantage préférée supérieure de 30 jours, à la durée pendant laquelle la concentration plasmatique après une administration unique parentérale, en particulier par voie parentérale non intraveineuse, des mêmes peptides ou protéines non préparé(e)s selon le procédé de préparation selon l'invention se trouve dans la fenêtre thérapeutique.

Dans un autre aspect, la composition pharmaceutique selon l'invention libère les protéines ou peptides selon l'invention après une administration unique parentérale, en particulier par voie parentérale non intraveineuse, de telle sorte que la concentration plasmatique en peptide ou protéine est supérieure à 85 %, de manière préférée supérieure à 90 %, de manière davantage préférée supérieure à 98 %, de la valeur de Cₘₐₓ entre Tₘₐₓ et T_{max+2 jours}, de préférence entre Tₘₐₓ et T_{max+7 jours}, de manière préférée entre Tₘₐₓ et Tₘₐₓ₊₂₁ jours, de manière plus préférée entre Tₘₐₓ et Tₘₐₓ₊₃₀ jours.

Avantageusement, la présente invention concerne une composition pharmaceutique comprenant des nanoprécipités ou nanocoprécipités selon l'invention de peptides ou protéines choisis parmi l'insuline humaine, l'hormone de croissance, le glucagon, les hormones peptidiques ou un de leurs dérivés ou fragments thérapeutiquement efficaces. Plus avantageusement la présente invention concerne une composition pharmaceutique comprenant des nanoprécipités selon l'invention d'insuline humaine ou d'un de ses dérivés ou fragments thérapeutiquement efficaces ou des nanocoprécipités selon l'invention d'insuline humaine ou d'un de ses dérivés ou fragments thérapeutiquement efficaces et d'ions métalliques, de préférence des nanocoprécipités d'insuline humaine / zinc.

Plus particulièrement, parmi les compositions adaptées à l'administration topique on trouve : les crèmes, les émulsions, les laits, les pommades, les lotions, les huiles, les solutions aqueuses ou hydro-alcooliques ou glycoliques, les poudres, les patchs, les sprays, les sticks, de patch ou tout autre produit pour application externe. Les préparations adaptées à une administration orale peuvent se présenter sous forme de gélules ou de capsules, notamment molles, en particulier de gélatine ou végétales, de poudre, de comprimés, notamment à avaler, à croquer, machable ou effervescent. Elles peuvent encore se présenter sous forme de liquide, d'émulsion, de crème, de pâte, de poudre, de gel ou de suspension. Enfin, les compositions adaptées à une administration par voie parentérale non intraveineuse, c'est-à-dire au moyen d'une injection sous cutanée, intradermique ou intramusculaire, se présentent notamment sous forme de suspension, de solution injectable ou d'implant. Les compositions selon l'invention peuvent également être associées à des dispositifs médicaux, tels que des stents.

Les modes d'administration, les posologies et les formes galéniques optimales de la composition pharmaceutique selon l'invention peuvent être déterminés selon les critères généralement pris en compte dans l'établissement d'un traitement pharmaceutique adapté à un sujet comme par exemple l'âge ou le poids corporel du patient, la gravité de son état général, la tolérance au traitement, les effets secondaires constatés.

L'excipient pharmaceutiquement acceptable est connu de l'Homme du Métier et est choisi selon le mode d'administration de la composition pharmaceutique. Ainsi, l'excipient pharmaceutiquement acceptable peut être choisi dans le groupe constitué par les agents diluants, de dispersion, mouillants, liants, désintégrants, les colorants, les lubrifiants, les agents solubilisants, les agents promoteurs d'absorption, les filmogènes, les agents gélifiants, et leurs mélanges.

A titre d'exemple, une composition solide sous forme de comprimés comprend un véhicule pharmaceutique tel que la gélatine, l'amidon, le lactose, le stéarate de magnésium, le talc, la gomme arabique ou analogues ; et éventuellement un enrobage de saccharose ou d'autres matières appropriées. De même, une préparation en gélules comprend notamment un diluant et des gélules molles ou dures. Une préparation sous forme de sirop ou d'élixir peut contenir l'ingrédient actif conjointement avec un édulcorant, un antiseptique, ainsi qu'un agent donnant du goût et un colorant approprié. Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants. Pour une administration rectale, les suppositoires sont préparés avec des liants fondant à la température rectale, par exemple du beurre de cacao ou des polyéthylèneglycols. Enfin, pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Avantageusement, la composition pharmaceutique selon l'invention comprend les nanoprécipités ou nanocoprécipités selon l'invention, de préférence d'insuline ou un de ses dérivés ou fragments thérapeutiquement efficaces ou de nanocoprécipités d'insuline et d'ions métalliques, de préférence d'insuline et de zinc, incorporés dans une matrice polymérique, qui est un système à libération prolongée et stable et suffisamment fluide pour être aisément injectable et stérilisable. Elle comprend notamment les polymères choisis parmi les polymères à base d'acide glycolique, d'acide lactique, d'acide pluronique, de polyéthylène glycol, de polysaccharides tels que l'acide hyaluronique, de polypeptides, les copolymères triblock PEO-PPO (poly(ethylene oxide) - poly(propylene oxide)), à base d'isopropylacrylamide, les matrices lipidiques, les dendrimères, ou leur mélange. Plus particulièrement, les nanoprécipités selon l'invention sont formulés au sein d'un gel d'acide hyaluronique.

Dans un aspect particulier, l'invention concerne en outre la composition pharmaceutique à libération prolongée selon l'invention, pour son utilisation en tant que médicament.

La présente invention concerne également des nanoprécipités ou nanocoprécipités selon l'invention ou une composition pharmaceutique selon l'invention, pour son utilisation dans les domaines de la santé humaine et animale, notamment en oncologie, dégénérescence tissulaire et osseuse, ophtalmologie, endocrinologie (dont diabète de type I et II), maladies cardiovasculaires, dermatologie, dermocosmétologie, infectiologie, parasitologie, cardiologie, immunologie, l'hépatologie, hématologie, gastrologie, entérologie, rhumatologie, blessures, pneumologie, gynécologie, oto-rhino-laryngologie, produits diagnostiques.

En outre, la présente invention concerne également l'utilisation de nanoprécipités ou nanocoprécipités selon l'invention ou d'une composition pharmaceutique selon l'invention, pour la préparation d'un médicament, en particulier destiné au traitement et/ou à la prévention d'une maladie dans les domaines de la santé humaine et animale, notamment en oncologie, dégénérescence tissulaire et osseuse, ophtalmologie, endocrinologie (dont diabète de type I et II), maladies cardiovasculaires, dermatologie, dermocosmétologie, infectiologie, parasitologie, cardiologie, immunologie, l'hépatologie, hématologie, gastrologie, entérologie, rhumatologie, blessures, pneumologie, gynécologie, oto-rhino-laryngologie, produits diagnostiques.

L'invention concerne également une méthode de traitement et/ou de prévention d'une maladie dans les domaines de santé humaine et animale, notamment en oncologie, dégénérescence tissulaire et osseuse, ophtalmologie, endocrinologie (dont diabète de type I et II), maladies cardiovasculaires, dermatologie, dermocosmétologie, infectiologie, parasitologie, cardiologie, immunologie, l'hépatologie, hématologie, gastrologie, entérologie, rhumatologie, blessures, pneumologie, gynécologie, oto-rhino-laryngologie, produits diagnostiques, chez un sujet en ayant besoin, comprenant l'administration au sujet d'une quantité thérapeutiquement efficace de nanoprécipités ou nanocoprécipités selon l'invention ou d'une composition pharmaceutique selon l'invention.

Plus particulièrement, la présente invention concerne des nanoprécipités d'insuline ou un de ses dérivés ou fragments thérapeutiquement efficaces ou des nanocoprécipités d'insuline et d'ions métalliques, de préférence d'insuline et de zinc, selon l'invention ou une composition pharmaceutique selon l'invention, pour son utilisation dans le traitement et/ou la prévention du diabète.

De manière avantageusement préférée, les nanoprécipités d'insuline ou nanocoprécipités insuline/ion métallique selon l'invention permettent la libération de l'insuline de manière prolongée après administration unique parentérale, en particulier par voie parentérale non intraveineuse, de telle sorte que la durée pendant laquelle la concentration plasmatique de l'insuline se trouve dans la fenêtre thérapeutique est supérieure à la durée pendant laquelle la concentration plasmatique après une administration unique parentérale de l'insuline non préparé(e)s selon le procédé de préparation selon se trouve dans la fenêtre thérapeutique.

En particulier, les nanoprécipités d'insuline ou nanocoprécipités insuline/ion métallique selon l'invention permettent la libération de l'insuline de manière prolongée après administration unique parentérale, en particulier par voie parentérale non intraveineuse, de telle sorte que la durée pendant laquelle la concentration plasmatique desdits peptides ou protéines se trouve dans la fenêtre thérapeutique est supérieure à 2 jours, de préférence supérieure à 7 jours, de manière préférée supérieure à 21 jours, de manière davantage préférée supérieure à 30 jours.

En particulier, les nanoprécipités d'insuline ou nanocoprécipités insuline/ion métallique selon l'invention permettent la libération de l'insuline de manière prolongée après administration unique parentérale, en particulier par voie parentérale non intraveineuse, de telle sorte que la durée pendant laquelle la concentration plasmatique desdits peptides ou protéines se trouve dans la fenêtre thérapeutique est supérieure de 2 jours, de préférence supérieure de 7 jours, de manière préférée supérieure de 21 jours, de manière davantage préférée supérieure de 30 jours, à la durée pendant laquelle la concentration plasmatique après une administration unique parentérale, en particulier par voie parentérale non intraveineuse, des mêmes peptides ou protéines non préparé(e)s selon le procédé de préparation selon l'invention se trouve dans la fenêtre thérapeutique.

Dans un autre aspect, les nanoprécipités d'insuline ou nanocoprécipités insuline/ion métallique selon l'invention permettent la libération de l'insuline de manière prolongée après administration unique parentérale, en particulier par voie parentérale non intraveineuse, de telle sorte que la concentration plasmatique en insuline est supérieure à 85 %, de manière préférée supérieure à 90 %, de manière davantage préférée supérieure à 98 %, de Cₘₐₓ entre Tₘₐₓ et Tₘₐₓ₊₂ jours, avantageusement entre Tₘₐₓ et Tₘₐₓ₊₅ jours, encore plus avantageusement entre Tₘₐₓ et T_{max+7 jours}.

Dans une mode de réalisation préféré, la présente invention concerne une composition pharmaceutique à libération prolongée de nanoprécipités d'insuline ou un de ses dérivés ou fragments thérapeutiquement efficaces ou de nanocoprécipités d'insuline et d'ions métalliques, de préférence d'insuline et de zinc, selon l'invention, pour son utilisation dans le traitement et/ou la prévention du diabète. De manière préférée, la composition pharmaceutique selon l'invention permet une libération prolongée in vivo de l'insuline de plus de 2 jours, avantageusement de plus de 5 jours, plus avantageusement de plus de 30 jours, encore plus avantageusement pendant 90 jours.

Avantageusement, la composition pharmaceutique selon l'invention permet la libération prolongée après une administration unique parentérale, en particulier par voie parentérale non intraveineuse, d'insuline ou un de ses dérivés ou fragments thérapeutiquement efficaces ou de complexes insuline/ion métallique, de préférence insuline/zinc, selon l'invention, de telle sorte que la durée pendant laquelle la concentration plasmatique de l'insuline se trouve dans la fenêtre thérapeutique est supérieure à la durée pendant laquelle la concentration plasmatique après une administration unique parentérale de l'insuline non préparé(e)s selon le procédé de préparation selon l'invention se trouve dans la fenêtre thérapeutique.

En particulier, la composition pharmaceutique selon l'invention permet la libération prolongée après une administration unique parentérale, en particulier par voie parentérale non intraveineuse, d'insuline ou un de ses dérivés ou fragments thérapeutiquement efficaces ou de complexes insuline/ion métallique, de préférence insuline/zinc, selon l'invention, de telle sorte que la durée pendant laquelle la concentration plasmatique desdits peptides ou protéines se trouve dans la fenêtre thérapeutique est supérieure à 2 jours, de préférence supérieure à 7 jours, de manière préférée supérieure à 21 jours, de manière davantage préférée supérieure à 30 jours.

En particulier, la composition pharmaceutique selon l'invention permet la libération prolongée après une administration unique parentérale, en particulier par voie parentérale non intraveineuse, d'insuline ou un de ses dérivés ou fragments thérapeutiquement efficaces ou de complexes insuline/ion métallique, de préférence insuline/zinc, selon l'invention, de telle sorte que la durée pendant laquelle la concentration plasmatique desdits peptides ou protéines se trouve dans la fenêtre thérapeutique est supérieure de 2 jours, de préférence supérieure de 7 jours, de manière préférée supérieure de 21 jours, de manière davantage préférée supérieure de 30 jours, à la durée pendant laquelle la concentration plasmatique après une administration unique parentérale, en particulier par voie parentérale non intraveineuse, des mêmes peptides ou protéines non préparé(e)s selon le procédé de préparation selon l'invention se trouve dans la fenêtre thérapeutique.

Dans un autre aspect, la composition pharmaceutique selon l'invention permet la libération prolongée après une administration unique parentérale, en particulier par voie parentérale non intraveineuse, d'insuline ou un de ses dérivés ou fragments thérapeutiquement efficaces ou de complexes insuline/ion métallique, de préférence insuline/zinc, selon l'invention, de telle sorte que la concentration plasmatique en insuline est supérieure à 85 %, de manière préférée supérieure à 90 %, de manière davantage préférée supérieure à 98 %, de de Cₘₐₓ entre Tₘₐₓ et Tₘₐₓ₊₂ jours, avantageusement entre Tₘₐₓ et Tₘₐₓ₊₅ jours, encore plus avantageusement entre Tₘₐₓ et Tₘₐₓ₊₇ jours,

Dans le cadre de la présente invention, on entend par diabète, une maladie chronique incurable causée par une carence ou un défaut d'utilisation de l'insuline entraînant un excès de sucre dans le sang. Il existe deux type de diabètes : le diabète de type 1 se caractérise par l'absence totale de la production d'insuline, le diabète de type 2 (diabète non insulinodépendant ou DNID) est un trouble du métabolisme du glucose caractérisé par une élévation du taux de glucose dans le sang, l'hyperglycémie.

La présente invention concerne également l'utilisation de nanoprécipités d'insuline ou d'un de ses dérivés ou fragments thérapeutiquement efficaces ou de complexes insuline/ion métallique, de préférence insuline/zinc, selon l'invention ou d'une composition pharmaceutique selon l'invention, pour la préparation d'un médicament destiné au traitement et/ou à la prévention du diabète.

En outre, l'invention concerne une méthode de traitement et/ou de prévention du diabète, chez un sujet en ayant besoin, comprenant l'administration au sujet d'une quantité thérapeutiquement efficace d'un nanoprécipité ou nanocoprécipité selon l'invention ou d'une composition pharmaceutique selon l'invention.

### DESCRIPTION DES FIGURES

**Figure 1****.** Rendement de la nanoprécipitation de l'insuline en fonction de la quantité d'insuline.
**Figure 2****.** Rendement de la nanocoprécipitation de l'insuline et de MnCl₂ en fonction de la quantité d'insuline et de MnCl₂ (condition (1) 0,12 mg de MnCl₂ ; condition (2) 0,25 mg de MnCl₂).
**Figure 3****.** Rendement de la nanocoprécipitation de l'insuline et de CaCl₂ en fonction de la quantité d'insuline et de CaCl₂ (condition (1) 0,12 mg de CaCl₂ ; condition (2) 0,25 mg de CaCl₂).
**Figure 4****.** Rendement de la nanocoprécipitation de l'insuline et de ZnCl₂ en fonction de la quantité d'insuline et de ZnCl₂ (condition (1) 0,12 mg de ZnCl₂ ; condition (2) 0,25 mg de ZnCl₂).
**Figure 5****.** Rendement de la nanocoprécipitation de l'insuline et de zinc en fonction de la concentration en zinc.
**Figure 6****.** Rendement de la nanoprécipitation, ou nanocoprécipitation avec 0,12 mg de ZnCl₂, de l'insuline en fonction de la température
**Figure 7**. Profil de libération de l'insuline à partir de nanoprécipité d'insuline avec du glycofurol (◆) ou du PEG 550 (■) selon l'invention, à partir d'un gel d'acide hyaluronique à 1%.
**Figure 8****.** Profil de libération de l'insuline à partir d'un nanocoprécipité d'insuline et d'ions métalliques selon l'invention préparé avec du glycofurol et du ZnCl₂ (●), du glycofurol et du MnCl₂ (◆), du PEG 550 et du ZnCl₂ (○) ou du PEG 550 et du MnCl₂ (0), à partir d'un gel d'acide hyaluronique en comparaison avec le profil de libération d'insuline sous forme native à partir d'un gel d'acide hyaluronique (■).
**Figure 9****.** Profil de libération de l'insuline en fonction de la présence ou non d'une étape de remise en suspension des nanoprécipités. Cette étape de mise en suspension est effectuée avant dispersion des nanoprécipités d'insuline au sein de la matrice d'acide hyaluronique. La condition sans dilution préalable (◆) correspond exclusivement à de l'insuline sous forme nanoprécipitée. La condition avec dilution préalable (■) correspondant à un mélange d'insuline nanoprécipitée et d'insuline en solution non précipitée.
**Figure 10****.** Distribution de taille des nanoprécipitées d'insuline obtenue par diffusion dynamique de la lumière.

Les Exemples qui suivent visent à illustrer la présente invention.

### EXEMPLES

### Exemple 1 : Préparation de nanoprécipités d'insuline

Dans un flacon, une solution de 0,468 mg d'insuline humaine (Umuline® rapide de chez Lilly à 100 UI/ml) est mise en contact avec une solution de PEG 550 (un non-solvant organique à l'insuline). La préparation obtenue est mélangée par agitation douce puis le flacon est placé dans un bain de glace pendant 30 minutes à 4 °C. Le mélange est ensuite centrifugé en utilisant une gamme de force centrifuge comprise entre 10000 et 50000 g. Enfin, les nanoprécipités sont récupérés et la quantité d'insuline est déterminée par HPLC.

Le rendement de nanoprécipitation est exprimé en pourcentage par rapport à la masse d'insuline introduite au départ.

Afin de mesurer l'influence de la quantité d'insuline sur le rendement de la nanoprécipitation, le procédé ci-dessus est répété avec une masse d'insuline de 0,936 mg, 1,404 mg, 1,874 mg et 2,340 mg, et le rendement est calculé pour chaque nanoprécipitation.

Les résultats sont exposés dans la Figure 1. On peut alors constater que plus la quantité d'insuline à précipiter est faible, plus le rendement de la nanoprécipitation est élevé.

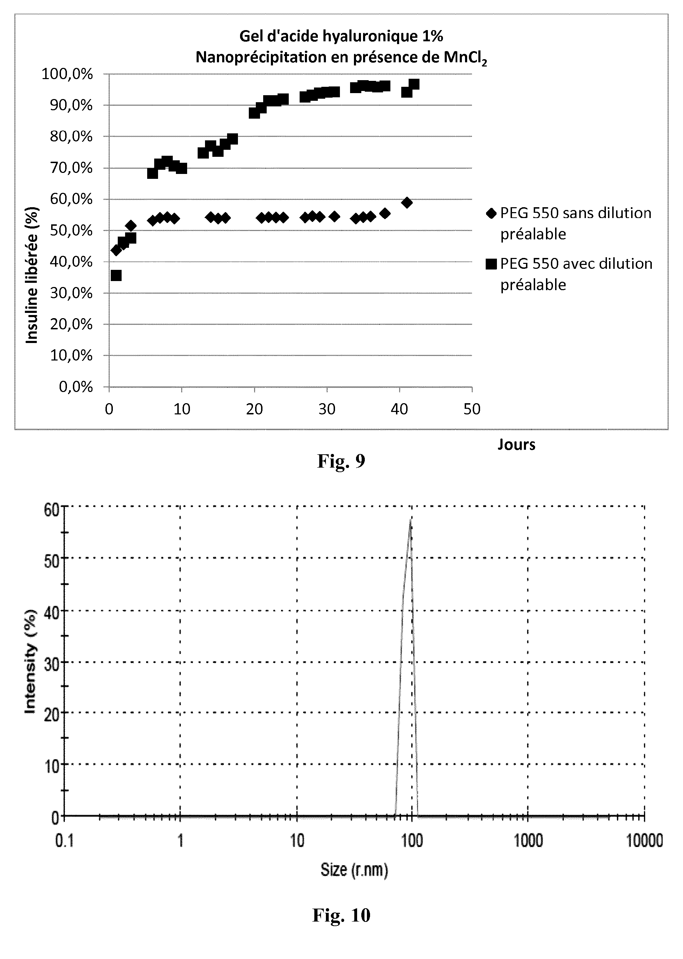

Le diamètre moyen des nanoprécipités obtenus est mesuré par diffusion dynamique de la lumière et est illustré par la Figure 10. On peut constater que ce diamètre moyen est inférieur à 200 nm.

### Exemple 2 : Influence de la présence d'un sel hydrosoluble lors de la nanoprécipitation

### Nanocoprécipitation d'insuline et de MnCl₂, CaCl₂ ou de ZnCl₂

Afin de mesurer l'influence de la présence d'un sel hydrosoluble sur le rendement de la nanoprécipitation d'insuline selon l'invention, en fonction de la quantité du sel et de la quantité d'insuline, trois sels ont été étudiés : le chlorure de manganèse MnCl₂, le chlorure de calcium CaCl₂ et le chlorure de zinc ZnCl₂. Pour chacun des sels, quatre solutions à nanoprécipiter ont été préparées selon les quantités données dans le tableau 1 ci-dessous :

**Tableau 1. Composition des solutions A, B C et D à nanoprécipiter**

| **Solution** | A | B | C | D |
|---|---|---|---|---|
| **Insuline humaine (mg)** | 0,135 | | 0,540 | |
| **PEG 550 (mg)** | 510 | | | |
| **Sel (mg)** | 0,12 | 0,25 | 0,12 | 0,25 |

Pour chacune des solutions, l'insuline humaine (Umuline® rapide de Eli Lilly à 100 UI/ml) est mise en contact avec le PEG 550 et le sel, dans les quantités présentées dans le tableau 1. La préparation obtenue est mélangée par agitation douce puis le flacon est placé dans un bain de glace pendant 30 minutes à 4 °C. Le mélange est ensuite centrifugé en utilisant une gamme de force centrifuge comprise entre 10000 et 50000 g. Enfin, les nanocoprécipités sont récupérés et la quantité d'insuline est déterminée par HPLC.

Le rendement de nanocoprécipitation est exprimé en pourcentage par rapport à la quantité d'insuline introduite au départ.

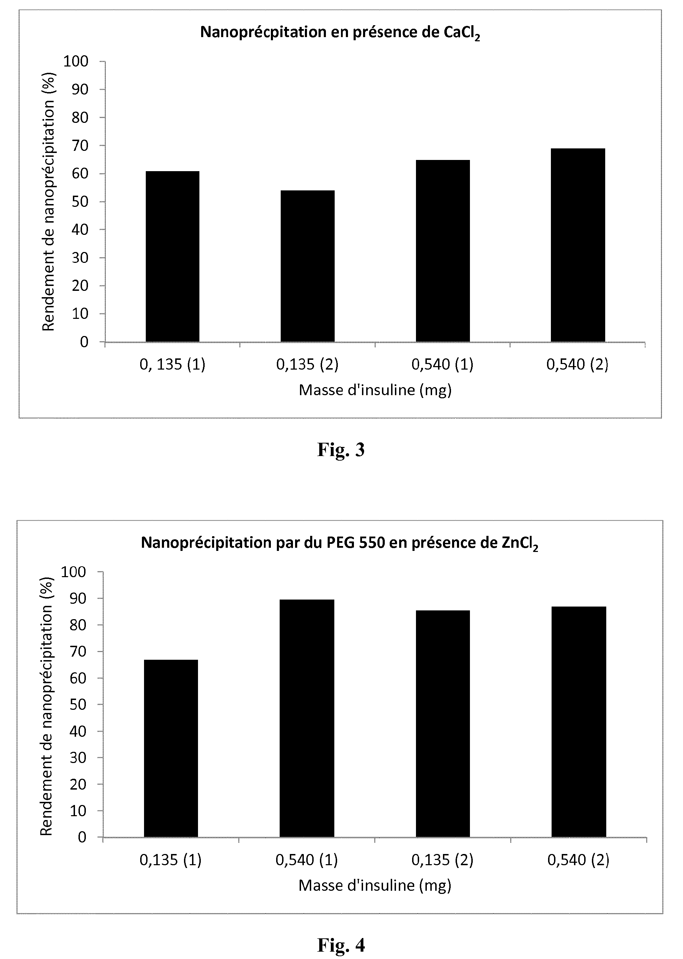

Les résultats sont exposés dans les Figures 2, 3 et 4 :
- à partir des Figures 2 et 3, on constate que l'utilisation de MnCl₂ ou de CaCl₂ permet d'obtenir des nanoprécipités d'insuline avec un rendement proche de 60-70% et ce quel que soit la quantité de MnCl₂, CaCl₂ ou d'insuline utilisée ;
- à partir de la Figure 4, on constate que l'utilisation de ZnCl₂ permet d'obtenir des nanoprécipités d'insuline avec un rendement proche de 80-90 % et ce quel que soit la quantité de MnCl₂ ou d'insuline utilisée.

L'utilisation de MnCl₂, CaCl₂ ou ZnCl₂, présente donc un double intérêt :
- maximisation du rendement de nanoprécipitation
- formation de nanocoprécipités ayant des propriétés différentes des nanoprécipités réalisés sans sels.

De manière générale, les Figures 2, 3 et 4 illustrent la souplesse du procédé selon l'invention vis-à-vis de la nature du sel utilisé. De plus, un même procédé (masse d'insuline, volume et nature du non-solvant identiques) permet d'obtenir des produits différents en termes de nature physico-chimiques et de propriétés biologiques en ne changeant que le sel.

### Nanocoprécipitation d'insuline et de ZnCl₂

Afin de compléter les résultats obtenus dans l'exemple 2, de nouvelles solutions sont préparées selon le procédé de l'exemple 2, dans les quantités données dans le tableau 2 ci-dessous :

**Tableau 2. Composition des solutions E, F, G, H, I, J, K, L, M, N, O, P à nanoprécipiter**

| **Solution** | E | F | G | H | I | J | K | L | M | N | O | P |
|---|---|---|---|---|---|---|---|---|---|---|---|---|
| **Insuline (mg)** | 0,936 | | | | 1,104 | | | | 1,872 | | | |
| **PEG 550 (mg)** | 510 | | | | | | | | | | | |
| **ZnCl2 (mg)** | 0 | 0,12 | 0,18 | 0,25 | 0 | 0,12 | 0,18 | 0,25 | 0 | 0,12 | 0,18 | 0,25 |

Le rendement de la nanocoprécipitation est exprimé en pourcentage par rapport à la masse d'insuline introduite au départ.

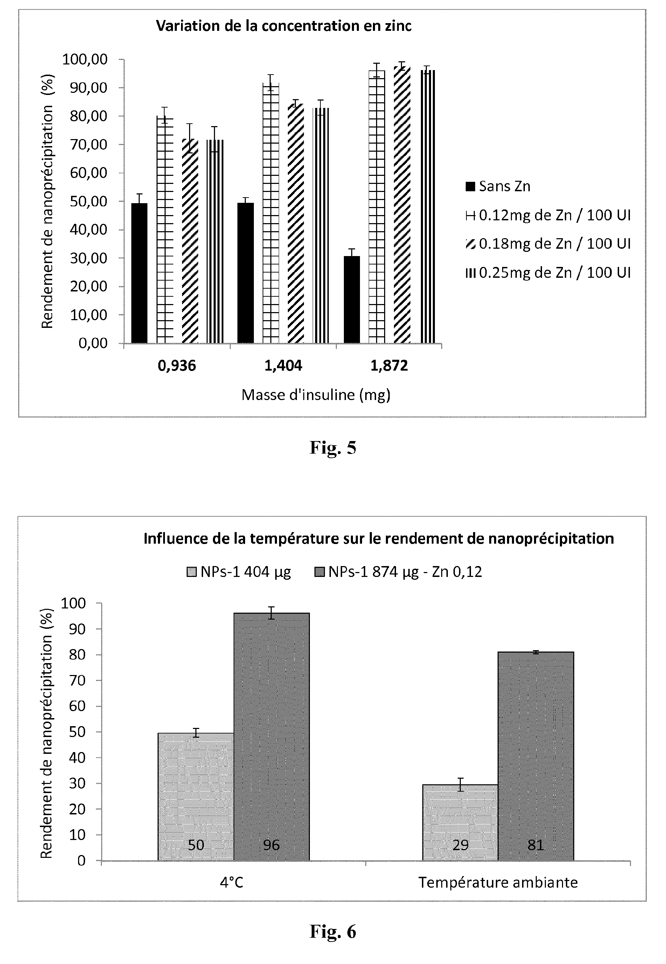

Les résultats sont exposés dans la Figure 5. A partir de ces résultats, il est à constater que les rendements les plus élevés sont obtenus pour les masses d'insuline les plus importantes. L'effet de la concentration en chlorure de zinc est donc négligeable.

Nous pouvons ainsi conclure que :
- le chlorure de zinc permet d'optimiser le rendement de nanoprécipitation ; les rendements obtenus étant proches de 100%.
- Le chlorure de zinc permet de former des nanocoprécipités ayant des propriétés spécifiques.

### Exemple 3 : Influence de la température sur le rendement de la nanoprécipitation ou nanocoprécipitation

Afin de mesurer l'influence de la température sur le rendement de la nanoprécipitation d'insuline selon l'invention en présence ou non d'un sel hydrosoluble, les rendements de la nanoprécipitation de 2 solutions différentes ont été étudiés pour deux conditions de températures différentes : à 4 °C et à température ambiante. La première solution est préparée selon le procédé décrit dans l'exemple 1 et comprend 1,404 mg d'insuline humaine (Umuline® rapide de chez Lilly à 100 UI/ml). La deuxième solution est préparée selon le procédé décrit dans l'exemple 2 et comprend 1,874 mg d'insuline humaine (Umuline® rapide de chez Lilly à 100 UI/ml) et 0.12 mg de chlorure de zinc. Le rendement de nanoprécipitation est exprimé en pourcentage par rapport à la masse d'insuline introduite au départ.

Les résultats sont exposés dans la Figure 6. Ils illustrent le fait que la nanoprécipitation peut être réalisée à température ambiante ce qui introduit deux notions importantes :
- souplesse du procédé selon l'invention et donc facilité d'utilisation et de mise à l'échelle
- différence notable avec les procédés décrits dans la littérature où la température est un facteur critique.

### Exemple 4 : Profil de libération d'insuline à partir de nanoprécipités ou nanocoprécipités d'insuline selon l'invention à partir d'un gel d'acide hyaluronique

Afin d'étudier le profil de libération d'insuline à partir de nanoprécipités d'insuline selon l'invention formulé au sein d'une matrice polymérique, ainsi que l'influence de la nature du non-solvant de l'insuline, les nanoprécipités d'insuline obtenus selon le procédé décrit dans l'exemple 1, en utilisant le PEG 550 ou le glycofurol en tant que non-solvant de l'insuline, sont formulés au sein d'un gel d'acide hyaluronique à 1%.

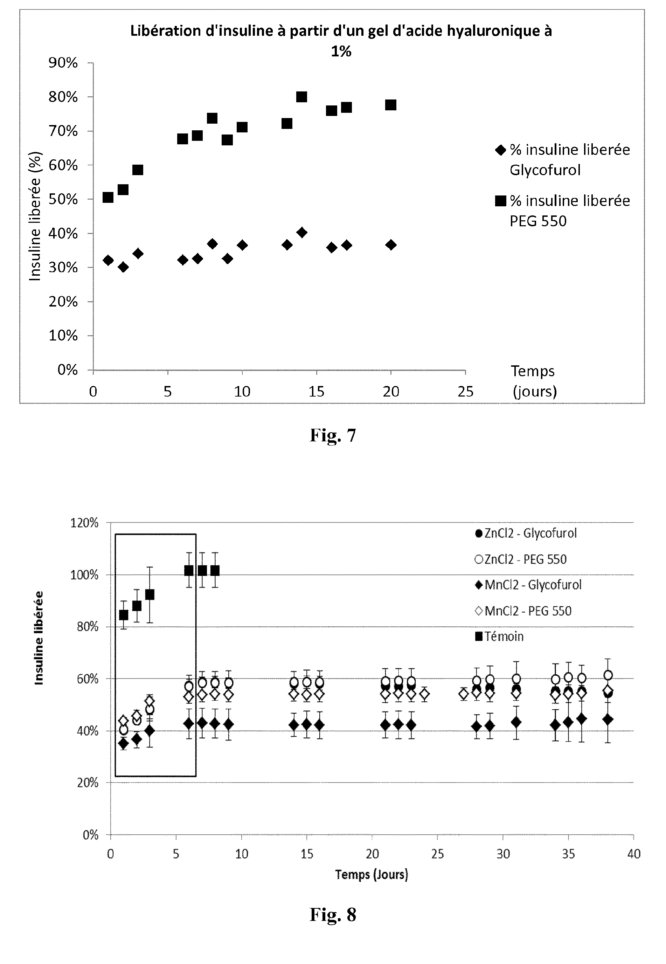

La Figure 7 représente la libération de l'insuline à partir desdits nanoprécipités d'insuline formulés au sein de la matrice de gel d'acide hyaluronique à 1 %. Ces résultats montrent que
- la libération se produit sur plus de 20 jours, et
- la nature du non-solvant influe sur la vitesse de libération ; l'utilisation de PEG 550 comme non-solvant de l'insuline permet d'obtenir une concentration plasmatique en insuline proche de 70 % sur plus de 20 jours, donc plus élevée que par l'utilisation de glycofurol (concentration plasmatique proche de 30-40 % sur plus de 20 jours).

De même, afin d'étudier le profil de libération d'insuline à partir de nanocoprécipité d'insuline et de sel hydrosoluble selon l'invention formulé au sein d'une matrice polymérique, ainsi que l'influence de la nature du non-solvant de l'insuline et du sel hydrosoluble, les nanocoprécipités d'insuline obtenus selon le procédé décrit dans l'exemple 2, en utilisant le PEG 550 ou le glycofurol en tant non-solvant de l'insuline, et du ZnCl₂ ou du MnCl₂ en tant que sel hydrosoluble, sont formulés au sein d'un gel d'acide hyaluronique à 1%.

Les profils de libération de l'insuline à partir desdits nanocoprécipités d'insuline et de sel hydrosoluble formulés au sein de la matrice de gel d'acide hyaluronique à 1 % sont observés dans la Figure 8 en comparaison avec le profil de libération d'insuline sous forme native. Ces résultats montrent que
- la libération de l'insuline à partir des nanocoprécipités se produit sur plus de 20 jours alors que la libération de l'insuline sous forme native est inférieur à 1 jour ;
- la nature du non-solvant influe sur la vitesse de libération. L'utilisation de PEG 550 permet d'obtenir une concentration plasmatique en insuline plus élevée que l'utilisation de glycofurol ; et
- la nature du sel hydrosoluble influe également sur la vitesse de libération. L'utilisation de ZnCl₂ permet d'obtenir une concentration plasmatique en insuline plus élevée que l'utilisation de MnCl₂.

### Exemple 5 : Profil de libération d'insuline en fonction de la présence ou non d'une étape de remise en suspension des nanocoprécipités selon l'invention.

Cette étude est effectuée afin de déterminer l'intérêt de la réversibilité du processus de nanoprécipitation ou nanocoprécipitation selon l'invention. En effet, lorsque les nanoprécipités ou nanocoprécipités selon l'invention sont mis en suspension en solution, ils reprennent leur forme native, c'est-à-dire non-précipités.

Pour cette étude, le profil de libération de l'insuline à partir de nanocoprécipités d'insuline et de MnCl₂ selon l'invention, formulés dans une matrice de gel d'acide hyaluronique (condition sans dilution), est comparé au profil de libération de ces mêmes nanocoprécipités mis en suspension en solution avant d'être formulés dans une matrice de gel d'acide hyaluronique (condition avec dilution). Les résultats sont observés dans la Figure 9.

La réversibilité de la nanoprécipitation ou nanocoprécipitation selon l'invention est un processus fondamental pour plusieurs points clés :
- la conservation de l'activité biologique de la protéine ou peptide ;
- la capacité des nanoprécipités à être une source in vivo de biothérapeutiques à partir de formes dépôts
- la capacité à moduler la vitesse de libération de la protéine ou peptide en jouant avec cette réversibilité (nanoprécipités, nanoprécipités remis en suspension partiellement, peptide ou protéine libre en solution).

## Revendications

1. Procédé de préparation non dénaturant de nanoprécipités de peptides ou protéines ou de nanocoprécipités de peptides ou protéines et d'ions métalliques, ayant un diamètre moyen inférieur à 1 µm, comprenant les étapes suivantes :
a) préparation d'un mélange d'une solution aqueuse de peptides ou protéines, d'un non-solvant des peptides ou protéines, et éventuellement un sel métallique hydrosoluble;
b) agitation douce du mélange obtenu à l'étape a) ;
c) séparation solide-liquide du mélange obtenu à l'étape b) ; et
d) éventuellement, récupération des nanoprécipités de peptides ou protéines ou de nanocoprécipités de peptides ou protéines et d'ions métalliques,
dans lequel lesdits peptides ou protéines sont de poids moléculaire inférieur à 15 kDa, avantageusement inférieur à 10 kDa, plus avantageusement inférieur à 8 kDa,
et ledit non-solvant est choisi parmi les polyéthylène glycols ou dérivés de polyéthylène glycols de poids moléculaire inférieur à 2000 Da, avantageusement compris entre 200 et 2000 Da, plus avantageusement de 550 Da, et les diols organiques choisis dans le groupe de l'hexylène glycol, butane-1,4-diol, pentane-1,5-diol, étohexadiol, 2-méthylpentane-2,4-diol(hexylène glycol), 3-cyclopentene-1,2-diol, cis-4-cyclopentene-1,3-diol, trans-1,4-dioxane-2,3-diol, 1,3-dioxane-5,5-dimethanol, (3S,4S)-pyrrolidine-3,4-diol, (3R,4R)-(-)-1-benzyl-3,4-pyrrolidinediol, (3S,4S)-(+)-1-benzyl-3,4-pyrrolidinediol, 3-cyclopentene-1,2-diol, 2-methyl-butane-1,3-diol.

2. Procédé selon la revendication 1, **caractérisé en ce que** le diamètre moyen des nanoprécipités ou nanocoprécipités est compris entre 5 nm et 500 nm, en particulier entre 5 et 200 nm, plus particulièrement entre 5 et 170 nm.

3. Procédé selon l'une quelconque des revendications 1 ou 2, **caractérisé en ce que** ledit ion métallique est choisi parmi les ions Zn, Mg, Ca, Mn, Fe, Li ou Cu, avantageusement ledit ion métallique est Zn ou Mn.

4. Procédé selon l'une quelconque des revendications précédentes, **caractérisé en ce que** ledit non-solvant est choisi parmi le PEG 550, le glycofurol ou l'hexylène glycol, en particulier parmi le PEG 550 et l'hexylène glycol, encore plus particulièrement ledit non-solvant est le PEG 550.

5. Procédé selon l'une quelconque des revendications précédentes, **caractérisé en ce que** lesdits peptides ou protéines sont thérapeutiques.

6. Procédé selon l'une quelconque des revendications précédentes, **caractérisé en ce que** lesdits peptides ou protéines sont choisi parmi l'insuline humaine, l'hormone de croissance, le glucagon, les hormones peptidiques ou un de leurs dérivés ou fragments thérapeutiquement efficaces.

7. Procédé selon l'une quelconque des revendications précédentes, **caractérisé en ce que** ledit sel hydrosoluble de métal est choisi parmi le ZnCl₂, MgCl₂, CaCl₂, MnCl₂, FeCl₂, LiCl et le CuSO₄, avantageusement le ZnCl₂ ou MnCl₂.

8. Procédé selon l'une quelconque des revendications précédentes, **caractérisé en ce que** ladite séparation solide/liquide de l'étape c) est une filtration tangentielle ou une centrifugation.

9. Nanoprécipités de peptides ou protéines ou nanocoprécipités de peptides ou protéines et d'ions métalliques, susceptibles d'être obtenus par le procédé de préparation selon l'une quelconque des revendications 1 à 8.

10. Nanoprécipités ou nanocoprécipités selon la revendication 9, **caractérisé en ce que** le peptide ou la protéine est l'insuline humaine ou un dérivé ou un fragment thérapeutiquement efficace.

11. Nanoprécipités ou nanocoprécipités selon les revendications 9 ou 10, pour leur utilisation en tant que médicament.

12. Nanoprécipités ou nanocoprécipités selon les revendications 9 ou 10, pour leur utilisation en tant que médicament à administration unique parentérale de telle sorte que la durée pendant laquelle la concentration plasmatique desdits peptides ou protéines se trouve dans la fenêtre thérapeutique est supérieure à la durée pendant laquelle la concentration plasmatique après une administration unique parentérale des mêmes peptides ou protéines non préparé(e)s selon le procédé de préparation tel que revendiqué dans les revendications 1 à 8 se trouve dans la fenêtre thérapeutique.

13. Composition pharmaceutique à libération prolongée comprenant des nanoprécipités ou nanocoprécipités selon l'une quelconque des revendications 9 à 12.

14. Composition pharmaceutique à libération prolongée selon la revendication 13, pour son utilisation en tant que médicament.

15. Composition pharmaceutique selon les revendications 13 ou 14, **caractérisée en ce que** le peptide ou la protéine est choisi parmi l'insuline humaine, l'hormone de croissance, le glucagon, les hormones peptidiques ou un de leurs dérivés ou fragments thérapeutiquement efficaces.
